(19) Europäisches Patentamt European Patent Office Office européen des brevets

(11) **EP 4 650 749 A1**

(12) **EUROPEAN PATENT APPLICATION**

(43) Date of publication:
**19.11.2025 Bulletin 2025/47**

(21) Application number: **25175636.7**

(22) Date of filing: **12.05.2025**

(51) International Patent Classification (IPC):
**G01N 15/00** (2024.01)  **G01N 15/04** (2006.01)
**G01N 15/10** (2024.01)

(52) Cooperative Patent Classification (CPC):
**G01N 15/04; G01N 15/10;** G01N 2015/0038;
G01N 2015/045; G01N 2015/1028;
G01N 2015/1029

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **13.05.2024  US 202463646471 P**

(71) Applicants:
• **National Institute of Health Sciences
Kawasaki-shi, Kanagawa 210-9501 (JP)**

• **SHIMADZU CORPORATION
Kyoto-shi, Kyoto 604-8511 (JP)**

(72) Inventors:
• **YAMAMOTO, Eiichi
Kawasaki-shi, Kanagawa 210-9501 (JP)**
• **NIKKO, Masataka
Kyoto-shi, Kyoto 604-8511 (JP)**
• **AOKI, Kengo
Kyoto-shi, Kyoto 604-8511 (JP)**

(74) Representative: **Müller-Boré & Partner
Patentanwälte PartG mbB
Friedenheimer Brücke 21
80639 München (DE)**

(54) **METHODS FOR DETECTING PARTICLES USING CENTRIFUGAL FIELD FLOW FRACTIONATION**

(57)    The present disclosure provides a method of detecting size, density, or size and density of the lipid nanoparticles. The detection may be performed by centrifugal field flow fractionation using a carrier solution comprising a monosaccharide.

FIGURE 1

EP 4 650 749 A1

**Description**

CROSS REFERENCE TO RELATED APPLICATIONS

This nonprovisional application is based on U.S. Provisional Application No. 63/646471 filed on May 13, 2024 with the U.S. Patent and Trademark Office, the entire contents of which are hereby incorporated by reference.

BACKGROUND OF THE INVENTION

Field of the Invention

The present disclosure includes methods for sorting particles by centrifugal field flow fractionation.

Description of the Background Art

Nanomedicine involves the use of nanotechnology to produce nanoparticles of active pharmaceutical ingredients or formulate nanoparticles for uses, such as in drug delivery. Accurately understanding the relationship between the physicochemical properties, such as particle sizes, morphologies, and surface properties, of nanoparticles and the corresponding biological responses is a major challenge. Particle size and particle size distribution are regarded as quality attributes of nanomedicines that need to be considered.

Currently, electron microscopy and dynamic light scattering ("DLS" hereinafter) are used to characterize the sizes and shapes of nanoparticles. DLS, in particular, is commonly applied to determine particle size distributions in the field of nanomedicine. Fractionation methods, such as field-flow fractionation ('FFF" hereinafter), using various fields, such as asymmetric flow, centrifugal, electrical, thermal, and magnetic, have recently received considerable attention for determining the accurate size distributions of nanoparticles. Among the FFF techniques, asymmetric flow-field flow fractionation ("AF4" hereinafter) provides high resolution in the presence of differently-sized particles in polydisperse mixtures, through fractionation of nanoparticles based on the diffusion coefficient (i.e., hydrodynamic size) of the particles. AF4 is being increasingly used to determine the sizes, stabilities, and drug-release capabilities of nanomedicines. By contrast, centrifugal FFF ("CF3" hereinafter), known as sedimentation field-flow fractionation, may separate particles based on their sizes as well as the difference in density between the particles and the elution fluid.

SUMMARY OF THE INVENTION

Evaluation based on particle size and density has not been attempted thus far in terms of the physicochemical properties of nanoparticles for use as nanomedicines. The evaluation of particle sizes and densities of nanoparticles may facilitate the sophisticated evaluation of their qualities and physicochemical properties.

In one aspect, disclosed herein is a method of detecting, profiling, sorting, and isolating nanoparticles based on the particle sizes and densities of nanoparticles used in nanomedicines using CF3. The nanoparticles may be lipid nanoparticles. Further disclosed herein are methods of administering lipid nanoparticles that are isolated and prepared using CF3. Further disclosed here are pharmaceutical compositions comprising lipid nanoparticle encapsulating a therapeutic agent and sorted and/or isolated using CF3.

The method of sorting lipid nanoparticles according to some embodiments may comprise sorting the lipid nanoparticles based on size, density, or size and density by CF3 using a carrier solution comprising a monosaccharide. The monosaccharide may be glucose. In some embodiments, the lipid nanoparticles may comprise a therapeutic agent, such as a messenger RNA molecule. The messenger RNA molecule may encode a vaccine. In other embodiments, the therapeutic agent encapsulated by the lipid nanoparticles may be an influenza vaccine, a cancer drug, or an RNAi drug. The carrier solution in some embodiments may be a phosphate buffer.

According to some embodiments, a method of detecting lipid nanoparticles may comprise sorting the lipid nanoparticles based on size, density, or size and density by CF3 using a carrier solution comprising a monosaccharide and detecting a lipid nanoparticle comprising a therapeutic agent. The detecting according to some embodiments may be performed using at least one selected from the group consisting of a multi-angle light scattering ("MALS" hereinafter) detector, a photodiode array detector, and an absorbance detector. The monosaccharide may be glucose. In some embodiments, the lipid nanoparticles may comprise a therapeutic agent, such as a messenger RNA molecule. The messenger RNA molecule may encode a vaccine. In other embodiments, the therapeutic agent encapsulated by the lipid nanoparticles may be an influenza vaccine, a cancer drug, or an RNAi drug. The carrier solution in some embodiments may be a phosphate buffer. The method may further comprise detecting a lipid nanoparticle not comprising a therapeutic agent. The method may comprise detecting agglutinated lipid nanoparticles.

According to some embodiments, a method of isolating lipid nanoparticles comprising a therapeutic agent may comprise separating the lipid nanoparticles based on size, density, or size and density by CF3 using a carrier solution comprising a monosaccharide, detecting a lipid nanoparticle comprising a therapeutic agent, and isolating the lipid nanoparticle comprising the therapeutic agent. The detecting according to some embodiments may be performed using at least one selected from the group consisting of a MALS detector, a photodiode array detector, and an absorbance detector. The monosaccharide may be glucose. In some embodiments, the lipid nanoparticles may comprise a therapeutic agent, such as a messenger RNA molecule. The messenger RNA molecule may encode a vaccine. In other embodiments, the therapeutic agent encapsulated by the lipid nanoparticles may be an influenza vaccine, a cancer drug, or an RNAi drug. The method may further comprise detecting a lipid nanoparticle not comprising a therapeutic agent and/or detecting agglutinated lipid nanoparticles. The method may further comprise discarding the lipid nanoparticles detected not to comprise a therapeutic agent and/or discarding agglutinated lipid nanoparticles. The lipid nanoparticle detected in the methods described herein may not have a contact, during the detecting, with an isotonic agent. The lipid nanoparticle detected in the methods described herein may not have a contact, during the detecting, with an isotonic agent that suppress the electrostatic interactions between the nanoparticles and reduce particle aggregation by reducing the interactions between water and the phospholipids. The lipid nanoparticle detected in the methods described herein may not have a contact, during the detecting, with sugar.

According to some embodiments, a method of administering lipid nanoparticles comprising a therapeutic agent may comprise separating the lipid nanoparticles based on size, density, or size and density by CF3 using a carrier solution comprising a monosaccharide, detecting a lipid nanoparticle comprising a therapeutic agent, isolating the lipid nanoparticle comprising the therapeutic agent, and administering the isolated lipid nanoparticle comprising the therapeutic agent. The detecting according to some embodiments may be performed using at least one selected from the group consisting of a MALS detector, a photodiode array detector, and an absorbance detector. The monosaccharide may be glucose. In some embodiments, the lipid nanoparticles may comprise a therapeutic agent, such as a messenger RNA molecule. The messenger RNA molecule may encode a vaccine. In other embodiments, the therapeutic agent encapsulated by the lipid nanoparticles may be an influenza vaccine, a cancer drug, or an RNAi drug. The carrier solution in some embodiments may be a phosphate buffer. The method may further comprise detecting a lipid nanoparticle not comprising a therapeutic agent and/or detecting agglutinated lipid nanoparticles. The method may further comprise discarding the lipid nanoparticles detected not to comprise a therapeutic agent and/or discarding agglutinated lipid nanoparticles.

According to some embodiments, a pharmaceutical composition comprising the isolated lipid nanoparticles comprising a therapeutic agent may be prepared according to any one of aforementioned methods. Moreover, according to other embodiments, a system for isolating lipid nanoparticles comprising a therapeutic agent may comprise a particle separator that separates the lipid nanoparticles based on size, density, or size and density by CF3 using a carrier solution comprising a monosaccharide, a particle detector that detects a lipid nanoparticle comprising a therapeutic agent, and a particle isolator that isolates the lipid nanoparticle comprising the therapeutic agent. The particle detector according to some embodiments may be performed using at least one selected from the group consisting of a MALS detector, a photodiode array detector, and an absorbance detector.

BRIEF DESCRIPTION OF THE DRAWINGS

FIGURE 1 depicts an exemplary overview of the CF3 analysis according to some embodiments of the disclosure.
FIGURE 2 depicts a schematic diagram of the CF3 used in examples.
FIGURE 3 depicts representative CF3 fractograms of the four lipid nanoparticle (LNP) samples under optimized conditions.

DESCRIPTION OF THE PREFERRED EMBODIMENTS

The present invention may be understood more readily by reference to the following detailed description of the preferred embodiments of the invention. However, although different components and methods are disclosed and described, it is to be understood that this invention is not limited to specific formulations, assemblies or configurations, conditions, or methods, as such may vary, and any modifications thereto and variations therein will be apparent to those skilled in the art. It is also to be understood that the terminology used herein is for the purpose of describing specific embodiments only and is not intended to be limiting.

In one aspect, the present disclosure provides a method of detecting nanoparticles. The nanoparticles herein may be lipid nanoparticles, and all methods using the lipid nanoparticles may be applied to other types of nanoparticles. In some embodiments, the nanoparticle described herein may comprise liposome. In some embodiments, the nanoparticle described herein may exclude liposome. In some embodiments, detecting, discovering, determining, measuring, evaluating, counting, and assessing particles are used interchangeably and include quantitative and/or qualitative determinations. In some embodiments, the detecting is performed using a sensor. In some embodiments, the detecting is performed using at least one selected from the group consisting of a MALS detector, a photodiode array detector, and an absorbance detector. The term "detect" herein may include determining physical attributes of the lipid nanoparticles, such as particle size and particle density but not limited thereto. In some embodiments, the detection wavelength from about 100 to about 900 nm, from about 190 to about 800 nm, about 200 to about 300 nm, or about 220 to about 240 nm is used. In some embodiments, the detection wavelength is from about 100, 110, 120, 130, 140, 150, 160, 170, 180, 190, 200, 210, 220, 230, 240, 250, 260, 270, 280, 290, 300, 310, 320, or 330 nm and/or to about 900, 800, 700, 600, 500, 400, 300, 290, 280, 270, 260, 250, 240 nm or less.

Here, "nanoparticles" are particles with a diameter of less than about 1,000 nm (1 $\mu$m). The nanoparticles may comprise various biodegradable or non-biodegradable polymers, lipids, phospholipids or metals. The lipid nanoparticle according to some embodiments may include (but are not limited to) phospholipids, triacylglycerols, cholesterol, cholesterol esters, fatty-acyl esters, and the like. In some embodiments, the nanoparticle may comprise a combination of these ingredients. For example, in a preferred embodiment, the lipid core may be made of cholesterol ester and triglyceride (e.g., castor oil), the phospholipid layer may be made of egg yolk phospholipid, and the surfactant coating layer may be made of sodium taurodeoxicholate and Poloxamer 188.

A. Phospholipids of the Nanoparticle

Phospholipids suitable for use in the nanoparticles include (but are not limited to) diacylglyceride structures and phosphosphingolipids. Diacylglycerides structures include phosphatidic acid (phosphatidate) (PA); phosphatidylethanolamine (cephalin) (PE), phosphatidylcholine (lecithin) (PC), phosphatidylserine (PS) and phosphoinositides. The phosphosphingolipids include ceramide phosphorylcholine (Sphingomyelin) (SPH), ceramide phosphorylethanolamine (Sphingomyelin) (Cer-PE) and ceramide phosphoryl lipid. The phospholipids suitable for use in the nanoparticles formulation include natural phospholipid derivatives and synthetic phospholipid derivatives. Natural phospholipid derivatives include egg PC, egg PG, soy PC, hydrogenated soy PC and sphingomyelin. Synthetic phospholipid derivatives include: phosphatidic acid; phosphatidylcholine; 1,2-didecanoyl-sn-glycero-3-phosphocholine (DDPC); 1,2-Dilauroyl-sn-glycero-3-phosphocholine (DLPC); 1,2-dimyristoyl-sn-glycero-3-phosphocholine (DMPC); 1,2-Dipalmitoyl-sn-glycero-3-phosphocholine (DPPC); 1,2-Distearoyl-sn-glycero-3-phosphocholine (DSPC); 1,2-Dioleoyl-sn-glycero-3 -phosphocholine (DOPC); 1-Palmitoyl-2-oleoyl-sn-glycero-3-phosphocholine(POPC); 1,2-Dierucoyl-sn-glycero-3-phosphocholine (DEPC); phosphatidylglycerol (PG); 1,2-Dimyristoyl-sn-glycero-3-phosphoglycerol (DMPG); 1,2-Dipalmitoyl-sn-glycero-3-phosphoglycerol (DPPG); 1,2-Distearoyl-sn-glycero-3-phosphoglycerol (DSPG); 1-palmitoyl-2-oleoyl-sn-glycero-3-phosphoglycerol (POPG); Phosphatidtylethanolamine (DMPE); 1,2-Dimyristoyl-sn-glycero-3-phosphoethanolamine; 1,2-dipalmitoyl-sn-glycero-3-phosphoethanolamine (DPPE); 1,2-Distearoyl-sn-glycero-3-phosphoethanolamine (DSPE); 1,2-Dioleoyl-sn-glycero-3-phosphoethanolamine (DOPE).

In an embodiment, phospholipids suitable for use in the nanoparticles comprise 1,2-dimyristoyl-sn-glycero-3-phosphocholine (DMPC); phosphatidyl glycerol (DMPG);1,2-distearoyl-sn-glycero-3-phosphocholine (DSPC): 1,2-distearoyl-sn-glycero-3-phosphoglycerol (DSPG); and egg PC. In one embodiment, the phospholipid comprises egg PC.

B. Triglycerides (Triacylglycerols) of the Nanoparticle

Triglycerides suitable for use in the nanoparticles formulation include (but are not limited to) triglycerides which are liquid at room temperature. Triglycerides suitable for use in the nanoparticles are selected from the group comprising canola oil, castor oil, chia seed oil, coconut oil, corn oil, cottonseed oil, olive oil, palm oil, peanut oil, safflower oil, sesame oil, soybean oil and others. Triglycerides also include mono-, di- and tri-acyl glycerols, where the fatty acids can be Mono-unsaturated fatty acid (palmitoleic acid, oleic acid, elaidic acid, gadoleic acid, eicosenoic acid, erucic acid and others), di-unsaturated fatty acid (linoleic acid, eicosadienoic acid, docosadienoic acid and others) and polyunsaturated fatty acids (linolenic acid, dihomo-$\gamma$-linolenic acid, eicosatrienoic acid, stearidonic acid, arachidonic acid, eicosatetraenoic acid, eicosapentaenoic acid, tetracosapentaenoic acid, docosahexaenoic acid and others). The di- and tri-acyl glycerols can contain or not identical fatty acids. Fractionated triglycerides, modified triglycerides, synthetic triglycerides, hydrogenated triglycerides and mixtures of triglycerides may also be used.

In embodiments, triglycerides suitable for use in the nanoparticles comprise castor oil, soy oil, coconut oil, and/or hydrogenated castor oil. In certain embodiments, the triglyceride of the nanoparticles is castor oil, and the therapeutic agent may be dissolved in this component within the nanoparticle core.

C. Cholesterol and Cholesterol Esters of the Nanoparticle

Cholesterol esters refer to cholesterol esterified with saturated fatty acid, including (but not limited to) myristic acid, palmitic acid, stearic acid, arachidic acid, lignoceric acid, and the like, or an unsaturated fatty acid, including but not limited to palmitoleic acid, oleic acid, vaccinic acid, linoleic acid, linolenic acid, arachidonic acid, eicosatrienoic acid, stearidonic acid, arachidonic acid, eicosatetraenoic acid, eicosapentaenoic acid, tetracosapentaenoic acid, docosahexaenoic acid and the like.

In some embodiments, the cholesterol ester of the nanoparticles is cholesteryl oleate. The cholesterol esters are located in the lipid core, whereas cholesterol is located in the phospholipid layer. Cholesterol is typically used in a proportion of between 0 and 4%, or in at least 0.1%, at least 0.5%, or at least 1%, and up to 3.9%, or up to 3.5%, or up to 3% of the nanoparticle components.

In some embodiments, the lipid nanoparticles have an average lipid concentration from about 60 to about 90 wt%, about 70 to about 90 wt%, about 50 to about 80 wt%, about 40 to about 60 wt%, about 80 to about 95 wt%, about 30 to about 80 wt%, about 60 to about 80 wt% or about 50 to about 70 wt% based on the lipid nanoparticles. In some embodiments, the lipid nanoparticles have an average lipid concentration from about 30, 35, 40, 45, 50, 55, 60, 65, 70, 75, 80, 85, 90 wt% or more and/or below about 100, 95, 90, 85, 80, 75, 70, 65, 60, 55 or 50 wt% based on the total lipid nanoparticle amount.

In some embodiments, the average density of the lipid nanoparticles is lower than that of the carrier solution described herein. In some embodiments, the lipid nanoparticles have an average density from about 0.5 to about 1.5 g/ml, from about 0.8 to about 1.1 g/ml, from 0.9 to about 1.1 g/ml, from about 0.5 to about 0.8 g/ml, from about 0.5 to about 1.0 g/ml, from about 0.3 to about 2.0 g/ml, or from about 0.7 to about 1.4 g/ml. In some embodiments, the lipid nanoparticles have an average density from about 0.3, 0.4, 0.5, 0.6, 0.7, 0.8, 0.9, 1.0, 1.1, 1.2 g/ml or more, and/or to about 2.0, 1.9, 1.8, 1.7, 1.6, 1.5, 1.4, 1.3, 1.2, 1.1, 1.0 g/ml or less. In some embodiments, the particles herein have an average diameter from about 20 to about 130 nm, from about 20 to about 50 nm, about 20 to about 150 nm, about 10 nm to about 200 nm, or about 5 nm to 300 nm. In some embodiments, the lipid nanoparticles have an average diameter from about 10, 20, 30, 40, 50, 60, 70, 80, 90, 100, 110, 120, 130, 140, 150, 160, 170, 180, 190, 200, 210, 220, 230, 240, 250 nm or more, and/or to about 1000, 900, 800, 700, 600, 500, 400, 300, 200 nm or less.

As used herein, the term "about" means modifying, for example, lengths of nucleotide sequences, degrees of errors, dimensions, the quantity of an ingredient in a composition, concentrations, volumes, densities, diameters, sizes, process temperature, process time, yields, flow rates, pressures, and like values, and ranges thereof, refers to variation in the numerical quantity that may occur, for example, through typical measuring and handling procedures used for making compounds, compositions, concentrates or use formulations; through inadvertent error in these procedures; through differences in the manufacture, source, or purity of starting materials or ingredients used to carry out the methods; and like considerations. The term "about" also encompasses amounts that differ due to aging of, for example, a composition, formulation, or cell culture with a particular initial concentration or mixture, and amounts that differ due to mixing or processing a composition or formulation with a particular initial concentration or mixture. Whether modified by the term "about" the claims appended hereto include equivalents to these quantities. The term "about" further may refer to a range of values that are similar to the stated reference value. In certain embodiments, the term "about" refers to a range of values that fall within 50, 25, 10, 9, 8, 7, 6, 5, 4, 3, 2, 1 percent or less of the stated reference value.

In some embodiments, the lipid nanoparticles herein may be physically separated or "sorted" using CF3 with one of the detection methods described herein. In some embodiments, the CF3 described herein may comprise a high-performance liquid chromatography (HPLC) that may utilize the pressure-driven flow of a mobile phase through a column packed with a stationary phase. Based on the physical attributes attained from such detection, it may be further determined whether a particular lipid nanoparticle comprises a therapeutic agent and/or whether the agglutinated with other nanoparticles. The lipid nanoparticles described herein may be agglutinated. The term "agglutinated" lipid nanoparticles as used in connection with lipid nanoparticles herein means a group of lipid nanoparticles that are adhered together such that they are no longer efficient carriers of the therapeutic agents contained therein. The "agglutinated" lipid particles according to some embodiments may comprise about 2, 3, 4, 5, 8, 10, 15, 20, 25, 30, 35, 40, 50, 100, 150, 200, 300, 500, 700, 900, or 1000 lipid nanoparticles. In other embodiments, the "agglutinated" lipid particles may have a diameter from about 30 nm, 50 nm, 100 nm, 200 nm, 500 nm, 1,000 nm, 2,000 nm, 5,000 nm, 10,000 nm, 20,000 nm, 50,000 nm or more, and/or to about 50 nm, 100 nm, 200 nm, 500 nm, 1,000 nm, 2,000 nm, 5,000 nm, 10,000 nm, 20,000 nm, 50,000 nm, 100,000 nm or less. Yet in other embodiments, the "agglutinated" lipid particles may be determined using a threshold value of a different physical attribute.

Herein, the term "and/or" used herein is defined to indicate any combination of the components. Moreover, the singular forms "a," "an," and "the" may further include plural referents unless the context clearly dictates otherwise.

In some embodiments, the lipid nanoparticles may comprise a therapeutic agent. As used herein, the terms "therapeutic agent," "active agent" or "active ingredient" means therapeutically useful amino acids, peptides, proteins, nucleic acids, including but not limited to polynucleotides, oligonucleotides, genes and the like, carbohydrates and lipids. In some embodiments, the therapeutic agent may include, for example, at least one selected from the group consisting of a protein, an enzyme, a polysaccharide, a polynucleotide, an organic compound, and an inorganic compound. The therapeutic agents according to some embodiments may include neurotrophic factors, growth factors, enzymes, antibodies, neurotransmitters, neuromodulators, antibiotics, antiviral agents, antifungal agents and chemotherapeutic agents, vaccines, RNAi drug, and the like. The therapeutic agents may include drugs, prodrugs, antibiotics, diagnostic substances, contrast agents and precursors that can be activated when the therapeutic agent is delivered to a target cell or tissue.

In some embodiments, a nanoparticle comprising a therapeutic agent may be a nanoparticle containing a therapeutic agent in an amount exceeding a threshold amount. A threshold amount may be an amount relative to the maximum capacity that may be encapsulated in the cavity of a given lipid nanoparticle. For example, the threshold amount may be about 5%, 10%, 15%, 20%, 25%, 30%, 35%, 40%, 45%, 50%, 55%, 60%, 65%, 70%, 75%, 80%, 85%, 90%, or 95% of the maximum capacity. In some embodiments, the nanoparticle described herein may have a therapeutic agent in a concentration from about 1 wt% to about 50 wt%, from about 10 wt% to about 80 wt%, from about 20 wt% to about 50 wt%, from about 30 wt% to about 50 wt%, or from about 1 wt% to about 10 wt% based on the nanoparticle. In some embodiments, the lipid nanoparticles have an average therapeutic agent concentration from about 1, 5, 10, 15, 20, 25, 30, 35, 40, 45, 50, 55, 60, 65, 70, 75, 80, 85, 90 wt% or more and/or to about 100, 95, 90, 85, 80, 75, 70, 65, 60, 55, 50, 45, 40, 35, 30, 25 wt% or below based on the total lipid nanoparticle amount.

The lipid nanoparticles may comprise a pharmaceutically acceptable carrier. As used herein, the term "pharmaceutically acceptable carrier" means a chemical composition or compound with which an active ingredient may be combined and which, following the combination, can be used to administer the active ingredient to a patient. In some embodiments, "pharmaceutically acceptable carrier" also includes, but is not limited to, one or more of the following: excipients, surface active agents, dispersing agents, inert diluents, granulating and disintegrating agents, binding agents, lubricating agents, sweetening agents, flavoring agents, coloring agents, preservatives, physiologically degradable compositions such as gelatin, aqueous vehicles and solvents, oily vehicles and solvents, suspending agents, dispersing or wetting agents, emulsifying agents, demulcents, buffers, salts, thickening agents, fillers, antioxidants, stabilizing agents, and pharmaceutically acceptable polymeric or hydrophobic materials.

In some embodiments, an effective amount of the therapeutic agent may be administered in a subject in need thereof. The subject may be human. As used herein, the term "administering" refers to the placement of the lipid nanoparticles loaded with therapeutic agent into a subject by a method or route which results in at least partial localization of the therapeutic agent(s) at a desired site. The nanoparticles with therapeutic agent(s) can be administered in any suitable form and by any appropriate route that results in effective treatment in the subject. As used herein, "an effective amount" refers to the amount sufficient to bring about a desired result in an experimental setting. A "therapeutically effective amount" or "therapeutic dose" refers to an amount sufficient to produce a therapeutic response or beneficial clinical result in a patient. For the methods according to some embodiments, the therapeutically effective amount or dose can be estimated initially from cell culture assays, then the dosage can be formulated for use in animal models so as to achieve a circulating concentration range that includes the IC50 as determined in cell culture. Such information can then be used to determine a useful dose in a subject. An effective amount of the therapeutic agent may treat a disease in the subject. The disease may be a viral disease and/or COVID-19. The effective treatment may be selected from the group consisting of antipyretics, anti-virals, Remdesivir, Oseltamivir, steroids, plasma including antibodies to COVID-19 of subjects that recovered from COVID-19, chloroquine, hydroxychloroquine, and a vaccine against COVID-19. In some embodiments, the therapeutic agent may comprise a messenger RNA molecule. In some embodiments, the messenger RNA molecule may encode a vaccine.

In another aspect, the method described herein may use CF3. The CF3 may use a carrier solution. In some embodiments, the carrier solution is alkaline. Further, the buffer in the carrier solution in some embodiments may be any conventional buffer that may maintain a slightly alkalinic pH, including but not limited to phosphate buffer, a tris buffer, an acetate buffer, a sulphate buffer, a citrate buffer, tartarate buffer, or borate buffer. The carrier solution may have a pH from about 6.5 to about 10, from about 6.9 to about 8.0, from about 7.0 to about 9.0, from about 6.5 to about 9.5, from about 7.0 to about 8.5, or from about 6.5 to about 8.5. In some embodiments, the pH may be from about 6.0, 6.2, 6.4, 6.6, 6.8, 7.0, 7.2, 7.4, 7.6, 7.8, 8.0, 8.2, 8.4, 8.6, 8.8, 9.0 or more and/or to about 10.0, 9.8, 9.6, 9.4, 9.2, 9.0, 8.8, 8.6, 8.4, 8.2, 8.0, 7.8, 7.6, 7.4, 7.2, 7.0 or below.

In some embodiments, the concentration of the buffer in the carrier solution may be from about 1 mmol/L to about 100 mmol/L, from about 1 mmol/L to about 10 mmol/L, from about 10 mmol/L to about 30 mmol/L, from about 5 mmol/L to about 15 mmol/L, from about 20 mmol/L to about 60 mmol/L, or from about 30 mmol/L to about 50 mmol/L. For example, the carrier solution may be a buffer solution having a phosphate concentration of more than about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 mmol/L and/or less than about 100, 90, 80, 70, 60, 50, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8 , 7, 6, or 5 mmol/L. Similarly, the solution may contain a buffer having a concentration of more than about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 mmol/L and/or less than about 100, 90, 80, 70, 60, 50, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8 , 7, 6, or 5 mmol/L. Moreover, in some embodiments, the monosaccharide concentration in the carrier solution may be from about 1 wt% to about 30 wt%, from about 10 wt% to about 20 wt%, from about 15 wt% to about 20 wt%, from about 10 wt% to about 16 wt%, from about 5 wt% to about 20 wt%, or from about 13 wt% to about 24 wt%. For example, the carrier solution may contain more than about 1, 2, 5, 8, 10, 11 12, 13, 14, 15, 16, 17, 17.5, 18, 19, or 20 wt% and/or less than about 30, 29, 28, 27, 26, 25, 24, 23, 22, 21, 20, 19, 18, 17, 16, 15, 14, 13, 12, 11, 10 wt% of monosaccharide. Similarly, the solution may contain more than 1, 2, 5, 8, 10, 11 12, 13, 14, 15, 16, 17, 17.5, 18, 19, or 20 wt% and less than 30, 29, 28, 27, 26, 25, 24, 23, 22, 21, 20, 19, 18, 17, 16, 15, 14, 13, 12, 11, or 10 wt% of a different monosaccharide.

In some embodiments, the carrier solution is run in the CF3 at a flow rate from about 0.2 mL/min to about 2.5 mL/min, from about 0.5 mL/min to about 1.8 mL/min, from about 0.7 mL/min to about 1.3 mL/min, or from about 0.8 mL/min to about 1.2 mL/min. In some embodiments, the flow rate is from about 0.2, 0.3, 0.4, 0.5, 0.6, 0.7, 0.8, 0.9, 1.0 mL/min or more, and/or to 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, 1.1 mL/min or less. In some embodiments, the carrier solution is run in a temperature from about 50 °C to about 70 °C. The temperature at which the carrier solution is made to run may be from 20 to 70 °C, from 20 to 50 °C, from 24 to 40 °C, from 24 to 30 °C, or from 50 to 70 °C. In some embodiments, the carrier solution is run in a temperature from about 20, 24, 30, 40, 50, 60 °C or more, and/or to 100, 90, 80, 70, 60, 50 °C or less.

The carrier solution may comprise a monosaccharide. The monosaccharide may comprise at least one selected from the group consisting of glucose, fructose, galactose, mannose, xylose, fucose, galactosamine, glucosamine, mannosamine, glacturonic acid, glucuronic, acid, iduronic acid, mannuronic acid, N-acetyl glactosamine, N-acetyl glucosamine, N-acetyl mannosamine, N-acetyl muramic acid, 2-keto-3-deoxy-D-glycero-D-galactonononic acid, N-acetyl neuraminic acid, or N-glycolyl neuraminic acid. In some embodiment, the monosaccharide may comprise glucose.

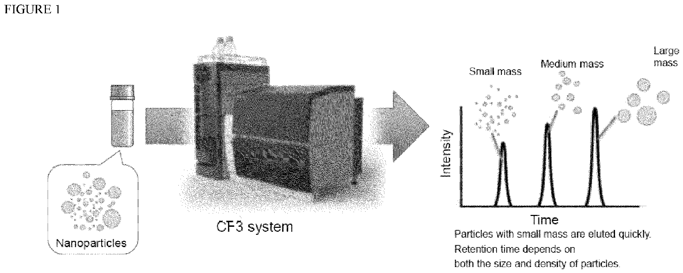

In some embodiments, the methods may comprise detecting size, density, or size and density of the particles. The size herein may include a diameter of the particles. Some embodiments of the present disclosure described herein utilizes CF3 to detect and/or sort lipid nanoparticles based on density and/or size. In CF3, increasing the applied centrifugal force on the particle may make it possible to separate smaller particles. In some embodiments, the CF3 may be a CF3 unit FFF-C8030 available from Shimadzu Corporation, which may apply a maximum centrifugal force of up to 12,000 rpm (156,000 m/s$^2$). A detailed explanation of CF3 may be found in, for example, U.S. Patent Application publication 2020/0001505, which is incorporated by reference in its entirety. An exemplary overview of the CF3 analysis according to some embodiments of the disclosure is illustrated in Fig. 1.

Some embodiments of the present disclosure use CF3 to determine size and/or density of the particles described herein to identify the particles that are loaded with a therapeutic agent.

In some embodiments, the methods described herein may sort the particles based on the size and/or density. The term "sort" as used in connection with the particles herein may comprise physically separating particles by CF3 based on physical features of the lipid nanoparticles, such as particle size, particle density, and other features that may serve as bases to group, classify, or categorize the particles.

In some embodiments, the methods described herein may isolate or purify the particles based on the size and/or density. The term "isolating" or "purifying" as used herein may comprise removing a group of lipid nanoparticles with a shared physical attribute from a pool of lipid nanoparticles containing another group having attributes different from the shared physical attribute. For example, in some embodiments, those lipid nanoparticles detected to comprise a therapeutic agent based on a certain physical attribute, such as a density value that falls within a predetermined range, may be separated from other lipid nanoparticles detected not to comprise a therapeutic agent based on a different physical attribute, such as a density value that falls outside of the aforementioned predetermined range. Isolated lipid nanoparticles may be further purified prior to administration to a subject. The term "isolating" or "purifying" as used herein may comprise removing or discarding a lipid nanoparticle that does not have a therapeutic agent.

In one aspect, the present disclosure also relates to a pharmaceutical composition prepared according to the methods described herein. The pharmaceutical composition may comprise a therapeutically effective amount of the therapeutic agent described herein. The pharmaceutical composition may comprise the pharmaceutically acceptable carrier described herein. In some embodiments, the pharmaceutical composition excludes isotonic agent that suppress the electrostatic interactions between the nanoparticles and reduce particle aggregation by reducing the interactions between water and the phospholipids. In some embodiments, the pharmaceutical composition may exclude sugar.

In one aspect, the present disclosure also relates to a method of administering the particles comprising a therapeutic agent as described herein. The method may treat a disease in a subject in need thereof. The subject may be human. The disease may be a viral disease. The disease may be COVID-19.

EXAMPLES

The embodiments of the disclosure are further explained in the following illustrative examples without limiting their scope.

The examples explained blow examines a profiling method based on the particle sizes and densities of nanoparticles used in nanomedicines using CF3 and a multiangle light scattering detector (CF3-MALS).

(Materials)

The Comirnaty® intramuscular injection (monovalent: original) (Pfizer), Comirnaty® intramuscular injection (bivalent: original/Omicron BA.1 and BA.4-5) (Pfizer), and Spikevax® divalent (bivalent: BA.4-5) (Moderna) were obtained from the Ministry of Health, Labor and Welfare in Japan. JIS Special Grade disodium hydrogen phosphate and sodium dihydrogen phosphate were purchased from FUJIFILM Wako Pure Chemical Industries (Osaka, Japan).

All other reagents were of special or analytical grade. Water was deionized and purified using a Milli-Q® TOC purification system (Millipore, Bedford, MA, USA).

(Preparation of lipid nanoparticle (LNP) samples)

The monovalent Comirnaty® injection was used as obtained after thawing and 1/5 dilution with phosphate-buffered saline (FUJIFILM Wako, Osaka, Japan). The bivalent Comirnaty® injection and Spikevax® were used as obtained after thawing according to the manufacturer's instructions.

(CF3)

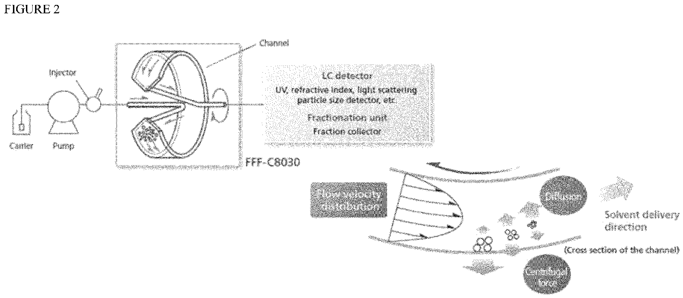

A schematic diagram of the CF3 used in this exemplary example is provided in Fig. 2. CF3 experiments were conducted using an FFF-C8030 (Shimadzu) equipped with a Nexera HPLC system (Shimadzu) and a rectangular stainless-steel channel with dimensions of 565.5 mm × 20 mm × 0.25 mm (length × breadth × height) and radius of rotation = 99 mm. The HPLC system comprised an LC-40D pump, DGU-405 degasser, SIL-40C autosampler, and SPD-M40A photodiode array detector (Shimadzu) or RF-20AXS fluorescence (FL) detector (Shimadzu). A Wyatt DAWN MALS detector (Waters, Milford, MA) was used for MALS detections. Instrument control and data evaluations were conducted using the Lab Solutions LC/GC (Shimadzu) and ASTRA (Wyatt Technology, California, U.S.) software. UV fractograms of LNPs were recorded in the wavelength range of 190-800 nm using an 8-nm slit, response rate of 1.28 s, sampling rate of 640 ms, and wavelength of 230 nm. The cell temperature was set at 40 °C. The angle-dependent scattering data used for MALS detection were evaluated using 14 active angles [light scattering (LS) 5-LS18]. The Berry model was used to fit the angular data obtained from the MALS detectors.

The flow rate of the carrier solution or eluent was 1.0 mL/min. The sample suspensions were placed in a Shimadzu TORAST-H glass vial (product number: 370-04301-01) and stored in an autosampler at 4 °C until the analysis. The carrier solution used for CF3 separation of the LNPs was 10 mmol/L phosphate buffer (pH 8.5), containing 18.4% glucose. The injection volume of the LNP samples containing Comirnaty® bivalent original/Omicron BA.1, Comirnaty® bivalent original/Omicron BA.4-5, and Spikevax® bivalent, was 10 μL. The injection volumes of the undiluted and diluted Comirnaty® original were 2 and 10 μL, respectively.

CF3 separation was conducted under the following conditions: an initial rotational speed of 12000 rpm (156,000 m/s$^2$), injection time of 1 min, relaxation time ($T_{relax}$) of 2.5 min, constant field period ($T_1$) of 10 min, and decay constant ($T_a$) of -120 min. The total analysis time

($T_{tot}$) was 120 min, and the rinse time ($T_{rinse}$) was 15 min.

(Example: CF3 analytical condition corresponding to the LNPs containing the COVID-19 vaccines)

The MALS detector was connected to acquire particle size data and fractograms. To determine the applicability of CF3 to LNP analysis, Spikevax®, an mRNA LNP, was chosen as the representative sample and analyzed with 10 mmol/L phosphate buffer as the carrier solution. The results revealed that much of the LNPs introduced to CF3 eluted with the void peak and it was difficult to retain them for CF3. Moreover, the recovery rate of the LNPs decreased to 72% at a neutral pH, and an improvement in recovery rate was observed at high pH values. For example, the recovery rate was 81% and 94% at pH 8.5 and 12, respectively. However, particle agglomeration was suspected as the pH of the solution increased, as per the MALS data. To improve the recovery rate of the LNPs from CF3 and to improve retention of LNPs, the carrier solution was investigated, and 10 mmol/L phosphate buffer (pH 8.5) containing 18.4% glucose was selected as the carrier solution. The actual pH of the carrier solution was 8. It was demonstrated that the addition of glucose to the phosphate buffer increased the density of the carrier solution and thereby significantly reduced the area of the void peak, making it possible to retain LNPs and properly carry out fractionation analysis by CF3. The cause of the improved retention may be expressed using Equation (1).

$$m_{eff} = m \frac{|\Delta_\rho|}{\rho_p} = m \frac{|\rho_p - \rho_l|}{\rho_p} \qquad (1)$$

The true mass (m) of a particle is related to its effective mass ($m_{eff}$) by its density ($\rho_p$) and the density of the carrier solution ($\rho_l$). Additionally, $\Delta_\rho$ is the difference in densities of the analyte and the carrier solution. The absolute value of $\Delta_\rho$ is used because the density of the analyte may be either higher or lower than that of the carrier solution.

In the examples above, the density ($\rho_l$) increased as a result of adding glucose to the phosphate buffer. Since it is considered that the value of density ($\rho_p$) is lower than the value of density ($\rho_l$) in LNP analysis with addition of glucose to the carrier solution, it is clearly understood that the increase of the density ($\rho_l$) caused increases of $\Delta_\rho$ and the effective mass ($m_{eff}$) and thereby the effect of improving LNP retention for CF3 analysis was attained.

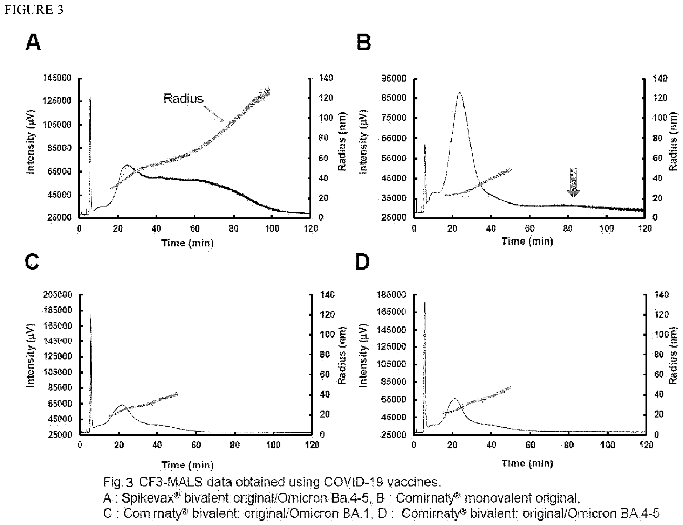

The analytical conditions were optimized using Spikevax® as the representative LNP sample. The recovery rates of the Spikevax®, Comirnaty® original, Comirnaty® bivalent original/Omicron BA.1, and Comirnaty® bivalent original/Omicron BA.4-5 samples from the CF3 system, calculated using the peak areas obtained from MALS detection, were 95%, 99%, 92%, and 97%, respectively. Diluting Comirnaty® original did not affect the elution profile in the fractograms obtained from CF3-MALS and the particle-size measurements via MALS. Fig. 3 shows the representative CF3 fractograms of the four LNP samples under optimized conditions.

The particle-size distributions of the four samples were in the ranges of 20-50 nm (radius) and 20-130 nm (radius). The CF3 fractograms of Sample A in Fig. 3 reveal that the 120-nm-radius particles eluted within approximately 100 min. A broad peak with low intensity was observed at approximately 100 min in the CF3 fractogram of Sample B, indicating the presence of particles similar to larger particles detected in Sample A, in this sample. Additionally, in Samples C and D, no peaks were detected at approximately 100 min in each CF3 fractogram, and the number of particles with a radius of approximately 100 nm was small.

(Discussion)

In the examples above, we employed CF3 to evaluate LNPs of various sizes and with different therapeutic agent contents (densities) to develop an analytical method for profiling injectable nanomedicines.

The difference in densities of the particle and carrier solution and centrifugal acceleration are two important parameters in CF3. Unlike liposomes, the LNPs may not possess a continuous bilayer around the mRNA-lipid matrix, which made them soft and limited their physical stability. The sample recovery rate problem from CF3 system may be solved by adding glucose to the carrier solution. The interaction between the LNPs and the channel surface may be attenuated. In addition to the liposome products, sugar is added as an isotonic agent to the COVID-19 vaccine formulations to suppress the electrostatic interactions between the nanoparticles and reduce particle aggregation by reducing the interactions between water and the phospholipids. Glucose is added to the carrier solution to reduce the interactions between the particles and the internal surface of the CF3 system, improving the retention of the LNPs by increasing the density of the carrier solution, which can make it possible to properly carry out CF3 analysis. The density of the LNPs was lower than that of the carrier solution employed in the examples above. The data obtained in the examples above suggests that CF3 combined with MALS is a valuable methodology for characterizing various nanoparticles based on particle sizes and densities.

In one aspect, the method described herein was developed for analyzing nanomedicines containing LNPs based on particle sizes and densities using CF3. The data obtained from the CF3 fractograms highlight that the proposed methodology may be used to characterize the physicochemical properties of LNPs. In some embodiments, the influence of particle density on *in vivo* dynamics may be addressed using CF3 to characterize nanoparticles in the future. Future therapeutic agent-delivery-system formulations with excellent efficacy may be developed through the proposed quantitative analysis of *in vitro* particle characteristics. In some embodiments, the present methods incorporate a wide range of information, including physicochemical and biological characteristics, such as particle-size distribution, *in vitro* and *in vivo* therapeutic agent release characteristics, *in vivo* particle diffusion, and stability. This may facilitate the development of innovative and high-quality nanomedicines for improving human health.

[Aspects]

As will be appreciated by those skilled in the art, the example embodiments and Examples described above are specific examples of the below aspects.

(Clause 1)

A method of detecting lipid nanoparticles, comprising:
detecting size, density, or size and density of the lipid nanoparticles by centrifugal field flow fractionation using a carrier solution comprising a monosaccharide.

(Clause 2)

The method according to Clause 1, wherein the lipid nanoparticles comprise a therapeutic agent.

(Clause 3)

The method according to Clause 1 or 2, wherein the lipid nanoparticles comprise a messenger RNA molecule.

(Clause 4)

The method according to any one of Clauses 1 to 3, wherein the lipid nanoparticles comprise a messenger RNA molecule encoding a vaccine.

(Clause 5)

The method according to any one of Clauses 1 to 4, wherein the lipid nanoparticles comprise at least one selected from the group consisting of phospholipids, triacylglycerols, cholesterol, cholesterol esters, fatty-acyl esters.

(Clause 6)

The method according to any one of Clauses 1 to 5, wherein the lipid nanoparticles have an average lipid concentration from 60% to 90% based on the lipid nanoparticles.

(Clause 7)

The method according to any one of Clauses 1 to 6, wherein the lipid nanoparticles have an average density from 0.5 g/mL to 1.5 g/mL.

(Clause 8)

The method according to any one of Clauses 1 to 7, wherein the lipid nanoparticles have an average diameter from 10 nm to 200 nm.

(Clause 9)

The method according to any one of Clauses 1 to 8, wherein the lipid nanoparticles comprise agglutinated lipid nanoparticles.

(Clause 10)

The method according to any one of Clauses 1 to 9, wherein the lipid nanoparticles comprise agglutinated lipid nanoparticles having an average diameter from 50 nm to 50,000 nm.

(Clause 11)

The method according to any one of Clauses 1 to 10, wherein the carrier solution comprises a phosphate buffer.

(Clause 12)

The method according to any one of Clauses 1 to 11, wherein the carrier solution has a phosphate concentration from 1 mmol/L to 100 mmol/L.

(Clause 13)

The method according to any one of Clauses 1 to 12, wherein the carrier solution is alkaline.

(Clause 14)

The method according to any one of Clauses 1 to 13, wherein the carrier solution has a monosaccharide concentration from 1 wt% to 30 wt%.

(Clause 15)

The method according to any one of Clauses 1 to 14, wherein the carrier solution has a pH from 7.0 to 9.5.

(Clause 16)

The method according to any one of Clauses 1 to 15 wherein the carrier solution has a pH from 7.5 to 9.0.

(Clause 17)

The method according to any one of Clauses 1 to 16, wherein the carrier solution is run in the centrifugal field flow fractionation at a flow rate from 0.5 mL/min to 1.8 mL/min.

(Clause 18)

The method according to any one of Clauses 1 to 17, wherein the carrier solution is run in a temperature from about 20 °C to about 70 °C.

(Clause 19)

The method according to any one of Clauses 1 to 18, wherein the monosaccharide comprises glucose.

(Clause 20)

The method according to any one of Clauses 1 to 19, wherein the centrifugal field flow fractionation comprises a high-performance liquid chromatography.

(Clause 21)

The method according to any one of Clauses 1 to 20, wherein the detecting is performed using at least one selected from the group consisting of a multi-angle light scattering (MALS) detector, a photodiode array detector, and an absorbance detector.

(Clause 22)

The method according to any one of Clauses 1 to 21, wherein the detecting is performed using a MALS detector.

(Clause 23)

The method according to any one of Clauses 1 to 22, wherein the detecting is performed with a detection wavelength from 190 to 800 nm.

(Clause 24)

The method according to any one of Clauses 1 to 23, wherein the detecting comprises detecting the size.

(Clause 25)

The method according to any one of Clauses 1 to 24, wherein the detecting comprises detecting the density.

(Clause 26)

The method according to any one of Clauses 1 to 25, further comprising determining whether the lipid nanoparticles contain a therapeutic agent of interest.

(Clause 27)

The method according to any one of Clauses 1 to 26, further comprising determining an amount of lipid nanoparticles that contain a therapeutic agent of interest.

(Clause 28)

A method of sorting or separating lipid nanoparticles, the method comprising

detecting the lipid nanoparticles according to the method of any one of Clauses 1 to 27, and
sorting the lipid nanoparticles based on the size, density, or size and density.

(Clause 29)

The method according to Clause 28, wherein the sorting results in a group of lipid nanoparticles sharing a target size and/or a target density of lipid nanoparticles.

(Clause 30)

A method of isolating or purifying lipid nanoparticles, the method comprising:

sorting or separating the lipid nanoparticles according to the method of Clause 28 or 29; and
isolating or purifying the lipid nanoparticle.

(Clause 31)

The method according to Clause 30, wherein the isolating or purifying comprises discarding a lipid nanoparticle that does not have a therapeutic agent.

(Clause 32)

The method according to Clause 30 or 31, wherein the isolating or purifying result in isolating or purifying a group of lipid nanoparticles sharing a target size and/or a target density of lipid nanoparticles.

(Clause 33)

The method according to Clause 30 or 31, wherein the isolating or purifying result in isolating or purifying a group of lipid nanoparticles containing a therapeutic agent.

(Clause 34)

A pharmaceutical composition prepared according to the method of any one of Clauses 30 to 33.

(Clause 35)

A method of administering lipid nanoparticles comprising a therapeutic agent, comprising

isolating or purifying the lipid nanoparticles according to the method of any one of Clauses 30 to 33; and administering the isolated lipid nanoparticle comprising the therapeutic agent.

(Clause 36)

A method of treating a disease in a subject in need thereof, comprising administering the lipid nanoparticles according to the method of Clause 35, wherein the lipid nanoparticles comprise a pharmaceutically effective amount of the therapeutic agent treating the disease.

(Clause 37)

The method according to Clause 36, wherein the disease comprises a viral disease.

(Clause 38)

The method according to Clause 36, wherein the disease comprises COVID-19.

(Clause 39)

Use of the product or method characterized by one or more elements disclosed in the application.

**Claims**

1. A method of detecting lipid nanoparticles, comprising:
   detecting size, density, or size and density of the lipid nanoparticles by centrifugal field flow fractionation using a carrier solution comprising a monosaccharide.

2. The method according to any one of the preceding claims, wherein the lipid nanoparticles comprise a messenger RNA molecule.

3. The method according to any one of the preceding claims, wherein the lipid nanoparticles comprise at least one selected from the group consisting of phospholipids, triacylglycerols, cholesterol, cholesterol esters, fatty-acyl esters.

4. The method according to any one of the preceding claims, wherein the lipid nanoparticles have an average lipid concentration from 60% to 90% based on the lipid nanoparticles.

**5.** The method according to any one of the preceding claims, wherein the lipid nanoparticles have an average density from 0.5 g/mL to 1.5 g/mL.

**6.** The method according to any one of the preceding claims, wherein the lipid nanoparticles have an average diameter from 10 nm to 200 nm.

**7.** The method according to any one of the preceding claims, wherein the lipid nanoparticles comprise agglutinated lipid nanoparticles having an average diameter from 50 nm to 50,000 nm.

**8.** The method according to any one of the preceding claims, wherein the carrier solution comprises a phosphate buffer.

**9.** The method according to any one of the preceding claims, wherein the carrier solution has a phosphate concentration from 1 mmol/L to 100 mmol/L.

**10.** The method according to any one of the preceding claims, wherein the carrier solution is alkaline.

**11.** The method according to any one of the preceding claims, wherein the carrier solution has a monosaccharide concentration from 1 wt% to 30 wt%.

**12.** The method according to any one of the preceding claims, wherein the carrier solution has a pH from 7.0 to 9.5.

**13.** The method according to any one of the preceding claims, wherein the carrier solution has a pH from 7.5 to 9.0.

**14.** The method according to any one of the preceding claims, wherein the carrier solution is run in the centrifugal field flow fractionation at a flow rate from 0.5 mL/min to 1.8 mL/min.

**15.** The method according to any one of the preceding claims, wherein the monosaccharide comprises glucose.

FIGURE 1

Particles with small mass are eluted quickly.
Retention time depends on
both the size and density of particles.

FIGURE 2

FIGURE 3

Fig.3 CF3-MALS data obtained using COVID-19 vaccines.
A : Spikevax® bivalent original/Omicron Ba.4-5, B : Comirnaty® monovalent original,
C : Comirnaty® bivalent: original/Omicron BA.1, D : Comirnaty® bivalent: original/Omicron BA.4-5

**EUROPEAN SEARCH REPORT**

Application Number

EP 25 17 5636

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/006414 A1 (CYTODIGM INC [US]) 4 January 2024 (2024-01-04) * pages 4-26; figures 1A-2B * | 1-15 | INV. G01N15/00 G01N15/04 G01N15/10 |
| A | US 2022/074927 A1 (LEEMAN MATS [SE] ET AL) 10 March 2022 (2022-03-10) * the whole document * | 1-15 | |

TECHNICAL FIELDS
SEARCHED       (IPC)

G01N

The present search report has been drawn up for all claims

| Place of search | Date of completion of the search | Examiner |
|---|---|---|
| The Hague | 19 September 2025 | Ionescu, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 5636

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2025

| Patent document cited in search report | | Publication date | Patent family member(s) | | Publication date |
|---|---|---|---|---|---|
| WO 2024006414 | A1 | 04-01-2024 | AU | 2023297939 A1 | 28-11-2024 |
| | | | CN | 119403544 A | 07-02-2025 |
| | | | EP | 4547224 A1 | 07-05-2025 |
| | | | JP | 2025525308 A | 05-08-2025 |
| | | | US | 2025001003 A1 | 02-01-2025 |
| | | | US | 2025099394 A1 | 27-03-2025 |
| | | | WO | 2024006414 A1 | 04-01-2024 |
| US 2022074927 | A1 | 10-03-2022 | DE | 20702333 T1 | 17-02-2022 |
| | | | EP | 3921836 A1 | 15-12-2021 |
| | | | US | 2022074927 A1 | 10-03-2022 |
| | | | WO | 2020161173 A1 | 13-08-2020 |

EPO FORM P0459

For more details about this annex : see Official Journal of the European Patent Office, No. 12/82

**EP 4 650 749 A1**

## REFERENCES CITED IN THE DESCRIPTION

**Patent documents cited in the description**

- US 63646471
- US 20200001505